Europäisches Patentamt
European Patent Office
Office européen des brevets

(19)

(11) Publication number: 0 408 806 A1

# (12) EUROPEAN PATENT APPLICATION

(21) Application number: 89201884.7

(22) Date of filing: 19.07.89

(51) Int. Cl.5: C07D 309/30, C07C 69/738, C07C 59/90, C07C 69/757

The applicant has filed a statement in accordance with Rule 28 (4) EPC (issue of a sample only to an expert). Accession numbers of the deposits: ATCC 35203 and ATCC 35204.

(43) Date of publication of application: 23.01.91 Bulletin 91/04

(84) Designated Contracting States: AT BE ES GR LU SE

(71) Applicant: MERCK & CO. INC.NC.
126, East Lincoln Avenue P.O. Box 2000
Rahway New Jersey 07065-0900(US)

(72) Inventor: Joshua, Henry
256 Woodward Avenue
Staten Island New York 10314(US)
Inventor: Schwartz, Michael S.
2045 Parkdale Avenue
Glenside, PA 19038(US)
Inventor: Wilson, Kenneth E.
120 Scotch Plains Avenue
Westfield New Jersey 07090(US)

(74) Representative: Hesketh, Alan, Dr. et al
European Patent Department Merck & Co., Inc. Terlings Park Eastwick Roadoad
Harlow Essex, CM20 2QR(GB)

## (54) Antihypercholesterolemic agents.

(57) Novel 3-hydroxy-3-methylglutaryl-coenzyme A (HMG-CoA) reductase inhibitors are useful as antihypercholesterolemic agents and are represented by the following general structural formulae (I) or (II):

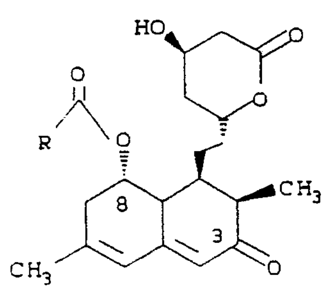

(I)

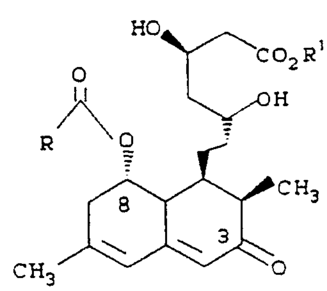

(II)

EP 0 408 806 A1

## ANTIHYPERCHOLESTEROLEMIC AGENTS

### BACKGROUND OF THE INVENTION

Hypercholesterolemia is known to be one of the prime risk factors for atherosclerosis and coronary heart disease, the leading cause of death and disability in Western countries.

There are agents known, however, that are very active antihypercholesterolemic agents which function by limiting cholesterol biosynthesis via inhibiting the enzyme, HMG-CoA reductase. These agents include the natural fermentation products, such as mevastatin, lovastatin and pravastatin, and semisynthetic analogs, such as simvastatin. These compounds have the following chemical structural formulae:

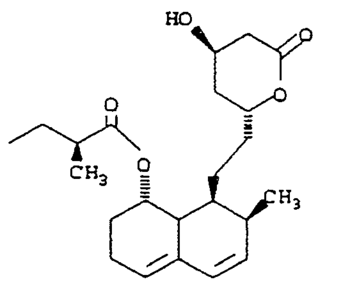

Mevastatin

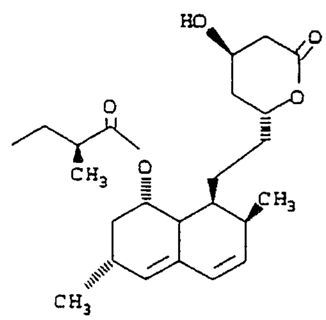

Lovastatin

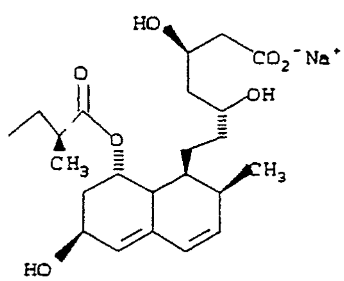

Pravastatin

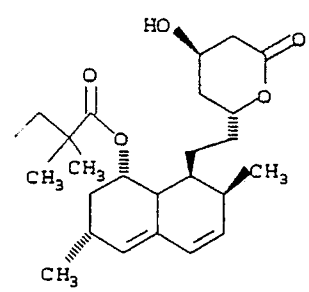

Simvastatin

Recently, MEVACOR®, which contains lovastatin as the active agent, was approved by the Food and Drug Administration for use as an antihypercholesterolemic drug.

Numerous analogs and homologs of these compounds have been described in the patent literature. U.S. Patent 4,444,784 discloses analogs of lovastatin which possess polyhydronaphthyl moieties and various 8-acyloxy groups attached thereto. U.S. Patent 4,444,784 also discloses numerous pharmaceutically acceptable salts of these analogs of lovastatin wherein the 8-acyloxy group has been elaborated broadly.

U.S. Patent 4,661,483 also discloses analogs of lovastatin wherein the 8-acyloxy group has been elaborated. Additionally, EP 245003, 245004 and 245990 disclose further analogs of lovastatin which have functionalized 8-acyloxy groups.

EP 251625 discloses compounds which are analogs of lovastatin and related compounds which possess a hydroxymethyl group or a carboxy group substituted on the 6-position of the polyhydronaphthyl moiety which are the microbiological oxidation products of lovastatin using the known strains of microorganism, Norcardia autotrophica, subspecies canberrica, ATCC 35203 of the culture MA-6181 and subspecies amethystina ATCC 35204 of the culture MA-6180.

EP European Application No. 88203032.8 discloses compounds which are analogs of lovastatin and related compounds which contain two double bonds in the 4,4a and 5,6 positions of the polyhydronaphthyl moiety.

## SUMMARY OF THE INVENTION

This invention relates to novel compounds which are HMG-CoA reductase inhibitors and are useful as antihypercholesterolemic agents. Specifically, the compounds of this invention are microbiological oxidation products of lovastatin and related compounds which possess a 1,2,7,8,8a(R)-pentahydronapthyl moiety and a 3-oxo group. Additionally, pharmaceutical compositions of these novel compounds, as a sole therapeutically active ingredient, and in combination with bile acid sequestrants are disclosed.

## DETAILED DESCRIPTION OF THE INVENTION

The specific HMG-CoA reductase inhibitors of this invention are the compounds represented by the following general structural formulae (I) and (II).

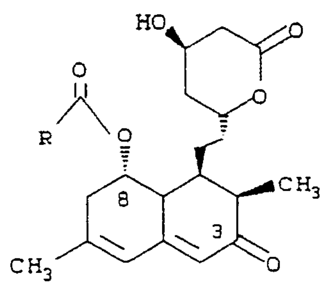

(I)

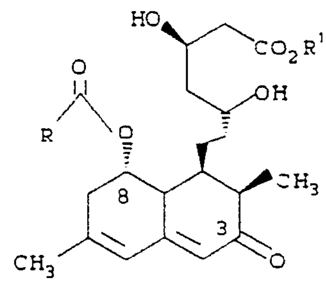

(II)

wherein:

R is $C_{1-10}$ alkyl;

$R^1$ is hydrogen, $C_{1-5}$alkyl, $C_{1-5}$ alkyl substituted with a member of the group consisting of phenyl, dimethylamino, or acetylamino;

and pharmaceutically acceptable salts of the compound (II) in which $R^1$ is hydrogen.

One embodiment of this invention are the compounds of the formulae (I) and (II) wherein R is sec -butyl or 1,1-dimethylpropyl and $R^1$ is hydrogen.

Illustrative of this embodiment is 6(R)-[2-[8(S)-(2-methylbutyryloxy)-2(S),6-dimethyl-3-oxo-1,2,7,8,8a(R)-pentahydronaphthyl-1(S)]ethyl]-4(R)-hydroxy-3,4,5,6-tetrahydro-2H-pyran-2-one; and 6(R)-[2-[8(S)-(2,2-dimethylbutyryloxy)-2(S),6-dimethyl-3-oxo-1,2,7,8,8a(R)-pentahydronaphthyl-1(S)]-ethyl]-4(R)-hydroxy-3,4,5,6-tetrahydro-2H-pyran-2-one.

Another embodiment of this invention is the class of compounds of the formula (II) wherein $R^1$ is $C_{1-5}$ alkyl and pharmaceutically acceptable salts of the compounds of the formula (II) wherein $R^1$ is hydrogen.

The pharmaceutically acceptable salts of this invention include those formed from cations such as sodium, potassium, aluminum, calcium, lithium, magnesium, zinc, and from bases such as ammonia, ethylenediamine, N methylglucamine, lysine, arginine, ornithine, choline, N,N′-dibenzylethylenediamine, chloroprocaine, diethanolamine, procaine, N-benzyl-phenethylamine, diethylamine, piperazine, tris-(hydroxymethyl)aminomethane, and tetramethylammonium hydroxide.

The compound of the formula (I) is conveniently prepared from lovastatin by one of three methods:

(a) adding the substrate to a growing culture of Nocardia autotrophica for a suitable incubation period followed by isolation, and derivatization if desired,

(b) collecting a culture of the bioconverting microorganism and contacting the collected cells with the substrate; or

(c) preparing a cell-free, enzyme-containing extract from the cells of the bioconverting microorganism and contacting this extract with the substrate.

Cultivation of the bioconverting microorganism of the genus Norcardia can be carried out by conventional means in a conventional culture medium containing nutrients well known for use with such microorganisms. Thus, as is well known, such culture media contain sources of assimilable carbon and of assimilable nitrogen and often inorganic salts. Examples of sources of assimilable carbon include glucose, sucrose, starch, glycerin, millet jelly, molasses and soybean oil Examples of sources of assimilable nitrogen

include soybean solids (including soybean meal and soybean flour), wheat germ, meat extracts, peptone, corn steep liquor, dried yeast and ammonium salts, such as ammonium sulphate. If required, inorganic salts, such as sodium chloride, potassium chloride, calcium carbonate or phosphates, may also be included. Also, if desired, other additives capable of promoting the production of hydroxylation enzymes may be employed in appropriate combinations. The particular cultivation technique is not critical to the process of the invention and any techniques conventionally used for the cultivation of microorganisms may equally be employed with the present invention. In general, of course, the techniques employed will be chosen having regard to industrial efficiency. Thus, liquid culture is generally preferred and the deep culture method is most convenient from the industrial point of view.

Cultivation will normally be carried out under aerobic conditions and at a temperature within the range from 20° to 37° C., more preferably from 26° to 28° C.

Method (a) is carried out by adding the substrate to the culture medium in the course of cultivation. The precise point during the cultivation at which the starting compound is added will vary depending upon the cultivation equipment, composition of the medium, temperature of the culture medium and other factors, but it is preferably at the time when the hydroxylation capacity of the microorganism begins to increase and this is usually 1 or 2 days after beginning cultivation of the microorganism. The amount of the substrate added is preferably from 0.01 to 5.0% by weight of the medium, more preferably from 0.05 to 0.5%, e.g., from 0.05 to 0.1% by weight. After addition of the substrate, cultivation is continued aerobically, normally at a temperature within the ranges proposed above. Cultivation is normally continued for a period of from 1 to 2 days after addition of the substrate.

In method (b), cultivation of the microorganism is first carried out under conditions such as to achieve its maximum hydroxylation capacity; this capacity usually reaches a maximum between 4 and 5 days after beginning the cultivation, although this period is variable, depending upon the nature and temperature of the medium, the species of microorganism and other factors. The hydroxylation capacity of the culture can be monitored by taking samples of the culture at suitable intervals, determining the hydroxylation capacity of the samples by contacting them with a substrate under standard conditions and determining the quantity of product obtained and plotting this capacity against time as a graph. When the hydroxylation capacity has reached its maximum point, cultivation is stopped and the microbial cells are collected. This may be achieved by subjecting the culture to centrifugal separation, filtration or similar known separation methods. The whole cells of the cultivating microorganism thus collected, preferably, are then washed with a suitable washing liquid, such as physiological saline or an appropriate buffer solution.

Contact of the collected cells of the microorganism of the genus Nocardia with the substrate is generally effected in an aqueous medium, for example in a phosphate buffer solution at a pH value of from 5 to 9. The reaction temperature is preferably within the range from 20° to 45° C., more preferably from 25° to 30° C. The concentration of the substrate in the reaction medium is preferably within the range from 0.01 to 5.0% by weight. The time allowed for the reaction is preferably from 1 to 5 days, although this may vary depending upon the concentration of the substrate in the reaction mixture, the reaction temperature, the hydroxylation capacity of the microorganism (which may, of course, vary from species to species and will also, as explained above, depend upon the cultivation time) and other factors.

The cell-free, enzyme-containing extract employed in method (c) may be obtained by breaking down the whole cells of the microorganism obtained as described in relation to method (b) by physical or chemical means, for example by grinding or ultrasonic treatment to provide a disintegrated cellular mass or by treatment with a surface active agent or an enzyme to produce a cellular solution. The resulting cell-free extract is then contacted with the substrate under the same conditions as are described above in relation to method (b).

The microorganism useful in the novel process of this invention is of the genus Nocardia . Of particular importance are the known strains of microorganism, Nocardia autotrophica , subspecies canberrica , ATCC 35203 of the culture MA-6181 and subspecies amethystina ATCC 35204 of the culture MA-6180 of the culture collection of Merck & Co., Inc., Rahway, New Jersey. A sample of the culture designated ATCC 35203 and ATCC 35204 is available in the permanent culture collection of the American Type Culture Collection at 12301 Parklawn Drive, Rockville, MD 20852.

After completion of the conversion reaction by any of the above methods, the desired compound can be directly isolated, separated or purified by conventional means. For example, separation and purification can be effected by filtering the reaction mixture, extraction the resulting filtrate with a water-immiscible organic solvent (such as ethyl acetate), distilling the solvent from the extract, subjecting the resulting crude compound to column chromatography, (for example on silica gel or alumina) and eluting the column with an appropriate eluent, especially in an HPLC apparatus.

The intrinsic HMG-CoA reductase inhibition activity of the claimed compounds is measured in the in

vitro protocol published in J. Med. Chem. , 28 , p. 347-358 (1985).

For estimation of relative inhibitory potencies, the $IC_{50}$ value of the test compound was compared with that of simvastatin determined simultaneously in the published in vitro protocol.

Representative of the intrinsic HMG-CoA reductase inhibitory activities of the claimed compounds is the relative potency of 6(R)-[2-[8(S)-(2,2-dimethylbutyryloxy)-2(S),6-dimethyl-3-oxo-1,2,7,8,8a(R)-pentahydronap hthyl-1(S)]ethyl]-4(R)-hydroxy-3,4,5,6-tetrahydro-2H-pyran 2-one which exhibited an $IC_{50}$ value of 2 ng/ml when compared to an $IC_{50}$ value of 4.2 ng/ml for simvastatin.

The compounds of this invention are useful as antihypercholesterolemic agents for the treatment of arteriosclerosis, hyperlipidemia, familial hypercholesterolemia and the like diseases in humans. They may be administered orally or parenterally in the form of a capsule, a tablet, an injectable preparation or the like. It is usually desirable to use the oral route. Doses may be varied, depending on the age, severity, body weight and other conditions of human patients but daily dosage for adults is within a range of from about 2 mg to 2000 mg (preferably 10 to 100 mg) which may be given in two to four divided doses. Higher doses may be favorably employed as required.

The compounds of this invention may also be coadministered with pharmaceutically acceptable nontoxic cationic polymers capable of binding bile acids in a non-reabsorbable form in the gastrointestinal tract. Examples of such polymers include cholestyramine, colestipol and poly[methyl-(3-trimethylaminopropyl)imino-trimethylene dihalide]. The relative amounts of the compounds of this invention and these polymers is between 1; 100 and 1.15,000.

Included within the scope of this invention is the method of treating arteriosclerosis, familial hypercholesterolemia or hyperlipidemia which comprises administering to a subject in need of such treatment a nontoxic, therapeutically effective amount of the compounds of formulae (I) or (II) or pharmaceutical compositions thereof.

The following examples illustrate the preparation of the compounds of the formulae (I) and (II) and their incorporation into pharmaceutical compositions and as such are not to be considered as limiting the invention set forth in the claims appended hereto.

## EXAMPLE 1

Preparation of 6(R)-[2-[8(S)-(2,2 dimethylbutyryloxy)-2(S),6-dimethyl-3-oxo-1,2,7,8,8a(R)-pentahydronaphthyl-1(S)]ethyl]-4(R)-hydroxy-3,4,5,6-tetrahydro-2H-pyran-2-one

Utilizing the general procedure for the bioconversion of sodium salt of 7-[1,2,6,7,8,8a(R)-hexahydro-2(S)-,6(R)-dimethyl-8(S)-(2,2 dimethylbutyryloxy)-1(S)-naphthyl]-3(R),5(R)-dihydroxy- heptanoic acid as described in EP 251625, the above titled compound was isolated as a minor product.

The following media are utilized in the bioconversion reactions described below.

| Medium A | Grams per liter distilled water |
|---|---|
| Yeast extract | 4.0 |
| Malt extract | 10.0 |
| Nutrient broth | 4.0 |
| Dextrose | 4.0 |
| pH 7.4<br>Medium sterilized for 20 min. at 121°C | |

| Medium B | Grams per liter distilled water |
|---|---|
| Dextrose | 10.0 |
| Polypeptone | 2.0 |
| Meat extract | 1.0 |
| Corn steep liquor | 3.0 |
| pH 7.0<br>Medium sterilized for 20 min. at 121°C | |

I. Culture Conditions and Bioconversion

A lyophilized tube of Nocardia autotrophica subsp. canberrica ATCC 35204 (MA-6180) was used to inoculate 18 x 175 agar slants (Medium A) which were incubated at 27°C for 7 days. The slant culture was washed with 5 ml of sterile medium B and transferred to a 250 ml flask containing 50 ml of sterile medium B. This first stage seed was grown at 27°C on a 220 rpm shaker and, after 24 hours, 2 ml was transferred to another flask of sterile medium B.

Grown under the above conditions, the second seed was used to start the bioconversion culture. 20 ml of the seed culture was placed in 400 ml of sterile medium B in a 2L flask. After the culture had grown for 24 hours, 80 mg of the sodium salt of 7-[1,2,6,7,8,8a(R)-hexahydro-2(S),6(R)-dimethyl-8(S)-(2,2-dimethylbutyryloxy)-1(S)-naphthyl]-3(R),5(R)-di-hydroxyheptanoic acid was added to each flask. The incubation was continued for 28 hours or until no 7-[1,2,6,7,8,8a(R)-hexahydro-2(S),6(R)-dimethyl-8(S)-(2,2-dimethylbutyryloxy)-1(S)-naphthyl]-3(R),5(R)-di-hydroxyheptanoic acid could be detected by HPLC. The whole broth was clarified by centrifugation followed by filtration through Whatman No. 2 filter paper.

II. HPLC Methods

Aliquots of whole broth could be analyzed for 7-[1,2,6,7,8,8a(R)-hexahydro-2(S),6(R)-dimethyl-8(S)-(2,2-dimethylbutyryloxy)-1(S)-naphthyl]-3(R),5(R)-dihydroxyheptanoic acid derivatives by HPLC. Filtered broth could be injected directly (10 to 20 µl) or after dilution with methanol. The compounds were separated on reversed phase columns utilizing a gradient of 35 to 45 percent aqueous acetonitrile at flow rates ranging between 1 and 3 ml/min. Addition of glacial acetic acid or $H_3PO_4$ (0.1 ml/L mobile phase) was required for the separation of the free acids. Derivatives of 7-[1,2,6,7,8,8a(R)-hexahydro-2(S),6(R)-dimethyl-8(S)-(2,2 dimethylbutyryloxy)-1(S)-naphthyl]-3-(R),5(R)-dihydroxyheptanoic acid were detected by monitoring the absorbance at 238 nm, as well as the absorbance ratio of 238 nm/228 nm. The desired products, 6(R)-[2-[8-(S)-(2-alkylacyloxy)-2(S),6-dimethyl-3-oxo-1,2,7,8,8a(R)-pentahydronaphthyl-1(S)]ethyl]-4(R) hydroxy-3,4,5,6-tetrahydro-2H-pyran-2-one were detected by monitoring the absorbance at 293 nm.

III. 6(R)-[2-[8(S)-(2,2-dimethylbutyryloxy)-2(S),6-dimethyl-3-oxo-1,2,7,8,8a(R)-pentahydronaphthyl-1(S)]-ethyl]-4(R)-hydroxy-3,4,5,6-tetrahydro-2H-pyran-2-one

Following the general procedure described above, the pH of the whole broth from the bioconversion of twenty kilograms of the sodium salt of 7-[1,2,6,7,8,8a(R)-hexahydro-2(S),6(R)-dimethyl-8(S)-(2,2-dimethylbutyryloxy)-1(S)-naphthyl]-3(R),5(R)-di-hydroxyheptanoic acid (12,700 liters) is adjusted to 4.0 with 2N sulfuric acid and is then extracted with ethyl acetate (2x4500 l.) The whole broth extraction is followed by an extraction into 1N sodium bicarbonate (20% by volume) and the aqueous extract is then washed with ethyl acetate. To the aqueous extract is then added methylisobutylketone (MIBK, 570 l.) and the pH of the aqueous phase adjusted to 3.1 using 7.2N sulfuric acid. The MIBK is then separated from the aqueous phase which is then extracted with MIBK (570 l.) and the MIBK extracts combined, filtered through diatomaceous earth, azeotropically dried and concentrated in vacuo to 870 liters. The MIBK solution is heated to 95°C, and then treated with trifluoroacetic acid (0.9 l.) in MIBK (23 l.). After about 15 minutes, the mixture is cooled to 25°C and washed successively with 1N sodium bicarbonate (0.5 volumes) and water (2x0.5 volumes). The organic phase is concentrated in vacuo and the residue dissolved in acetonitrile, which is then diluted to 30% acetonitrile using 0.02M phosphate buffer at pH=7. Aliquots (1/3) which contain approximately 700 gm. of 6(R)-[2-[8(S)-(2,2-dimethylbutyryloxy)-6-hydroxymethyl-2(S)-methyl-1,2,6,7,8,8a-(R)-hexahydronaphthyl-1(S)]ethyl]-4(R)-hydroxy-3,4,5,6-tetrahydro-2H-pyran-2-one were chromatographed over SP-207 (300 l, brominated copolymer of styrene and divinylbenzene, Mitsubishi Co.) column eluted with acetonitrile/buffer (30%, 37%, 47%, 57%,) and acetonitrile/water (67%) gave the above titled product and the 6-hydroxymethyl compound. The desired product may be further purified by removing most of the 6-hydroxymethyl compound by crystallization by dissolving the mixture in isopropyl acetate (IPAC) or methyl-t -butyl ether (MTBE) and then adding the solution to a non-polar solvent ( n -heptane, cyclohexane or petroleum ether).

IV. Isolation of 6(R)-[2-[8(S)-(2,2 dimethylbutyryloxy)-2(S),6-dimethyl-3-oxo-1,2,7,8,8a(R)-pentahydronaphthyl-1(S)]ethyl]-4(R)-hydroxy-3,4,5,6-tetrahydro-2H-pyran-2-one

The crystallization mother liquors from Step III were concentrated to an oil and then dissolved in toluene:methanol:acetonitrile (8:1:1, V:V:V) to a final volume of 100 ml. This solution was charged to a 10 liter columr of Sephadex LH-20 (Pharmacia Inc.) equilibrated with hexane:toluene:methanol (3:1:1, V:V:V) and eluted with this solvent at a flow rate of 100 ml/min.

The desired compound eluted between 11 and 14 column volumes and the eluant concentrated to a solid. The product was further purified by preparative reverse phase hplc on a $C_{18}$ column (21. 4 mm ID x 30 cm) eluted with a linear gradient starting 10 minutes after injection from 25% acetonitrile in water to 75% acetonitrile in water over 40 minutes at a flow rate of 10 ml/ min. The fractions containing the desired product (eluting at 29 minutes) were combined and concentrated to yield about 400 mg of the desired product in crystalline form. $^{13}C$ NMR Data ($CD_2Cl_2$, $\delta_c = 53.8$ ppm)

| ppm | ppm | ppm |
|---|---|---|
| 9.4 | 36.5 | 67.0 |
| 10.6 | 36.8 | 76.0 |
| 24.1 | 37.7 | 123.1 |
| 24.3 | 39.0 | 124.5 |
| 24.4 | 39.6 | 144.3 |
| 24.9 | 42.7 | 154.9 |
| 32.9 | 43.3 | 170.2 |
| 33.4 | 63.1 | 177.6 |
| | | 203.4 |

MS analysis showed a weak $M^+$ ion at m/z 432 and fragment ions at m/z 316 and 173 (base). UV spectrum exhibited a $\lambda_{max}$ = 290 nm, with $\epsilon = 21,900$.

In a similar fashion Nocardia autotrophica subsp. canberrica ATCC 35203 (MA6181) was utilized in the bioconversion reaction with the sodium salt of 7-[1,2,6,7,8,8a(R)-hexahydro-2(S),6(R)-dimethyl-8(S)-(2,2-dimethylbutyryloxy)-1(S)-naphthyl]-3(R),5(R)-di-hydroxyheptanoic acid to afford the desired products.

Additionally, the sodium salt of 7-[1,2,6,7,8,8a(R)-hexahydro-2(S),6(R)-dimethyl-8(S)-(2-methylbutyryloxy)-1(S)-naphthyl]-3(R),5(R)-dihydroxyheptanoic acid, the sodium salt of ring opened lovastatin, was subjected to analogous bioconversion reactions utilizing both N. autotrophica subsp. amethystina ATCC

35204 (MA6180) and N. autotrophic subsp. canberrica ATCC 35203 (MA6181) to afford 6(R)-[2-[8(S)-(2-methylbutyryloxy)-2(S),6-dimethyl-3-oxo-1,2,7,8,8a(R)-pentahydronaphthyl-1(S)]-ethyl]-4(R)-hydroxy-3,4,5,6-tetrahydro-2H-pyran-2-one.

## EXAMPLE 2

### Preparation of Ammonium Salts of Compounds II

The lactone (1.0 mmol) from Example 1 is dissolved with stirring in 0.1N NaOH (1.1 mmol) at ambient temperature. The resulting solution is cooled and acidified by the dropwise addition of 1N HCl. The resulting mixture is extracted with diethyl ether and the extract washed with brine and dried ($MgSO_4$). The $MgSO_4$ is removed by filtration and the filtrate saturated with ammonia (gas) to give a gum which solidified to provide the ammonium salt.

## EXAMPLE 3

### Preparation of Alkali and Alkaline Earth Salts of Compounds II

To a solution of 42 mg of lactone from Example 1 in 2 ml of ethanol is added 1 ml of aqueous NaOH (1 equivalent) After one hour at room temperature, the mixture is taken to dryness in vacuo to yield the desired sodium salt.

In like manner, the potassium salt is prepared using one equivalent of potassium hydroxide, and the calcium salt, using one equivalent of CaO.

## EXAMPLE 4

### Preparation of Ethylenediamine Salts of Compounds II

To a solution of 0.50 g of the ammonium salt from Example 2 in 10 ml of methanol is added 0.75 ml of ethylenediamine. The methanol is stripped off under vacuum to obtain the desired ethylenediamine salt.

## EXAMPLE 5

### Preparation of Tris(hydroxymethyl)aminomethane Salts of Compounds II

To a solution of 202 mg of the ammonium salt from Example 2 in 5 ml of methanol is added a solution of 60.5 mg of tris(hydroxymethyl) aminomethane in 5 ml of methanol. The solvent is removed in vacuo to afford the desired tris(hydroxymethyl)aminomethane salt.

## EXAMPLE 6

### Preparation of L-Lysine Salts of Compounds II

A solution of 0.001 mole of L-lysine and 0.0011 mole of the ammonium salt from Example 2 in 15 ml of 85% ethanol is concentrated to dryness in vacuo to give the desired L-lysine salt.

Similarly prepared are the L-arginine, L-ornithine, and N-methylglucamine salts.

## EXAMPLE 7

### Preparation of Tetramethylammonium Salts of Compounds II

A mixture of 68 mg of ammonium salt from Example 2 in 2 ml of methylene chloride and 0.08 ml of 24% tetramethylammonium hydroxide in methanol is diluted with ether to yield the desired tetramethylammonium salt

## EXAMPLE 8

### Preparation of Methyl Esters of Compounds II

To a solution of 400 mg of lactone from Example 1 in 100 ml of absolute methanol is added 10 ml 0.1 M sodium methoxide in absolute methanol. This solution is allowed to stand at room temperature for one hour, then is diluted with water and extracted twice with ethyl acetate. The organic phase is separated, dried ($Na_2SO_4$), filtered and evaporated in vacuo to yield the desired methyl ester.

In like manner, by the use of equivalent amounts of propanol, butanol, isobutanol, t-butanol, amylalcohol, isoamylalcohol, 2-dimethylaminoethanol, benzylalcohol, 2-acetamidoethanol and the like, the corresponding esters are obtained.

## EXAMPLE 9

### Preparation of Free Dihydroxy Acids

The sodium salt of the compound II from Example 3 is dissolved in 2 ml of ethanol-water (1:1; v:v) and added to 10 ml of 1N hydrochloric acid from which the dihydroxy acid is extracted with ethyl acetate. The organic extract is washed once with water, dried ($Na_2SO_4$), and evaporated in vacuo with a bath temperature not exceeding 30°C. The dihydroxy acid derivative slowly reverts to the corresponding, parent lactone on standing, but is stable at a pH above 7.

## EXAMPLE 10

As a specific embodiment of a composition of this invention 20 mg of lactone from Example 1, is formulated with sufficient finely divided lactose to provide a total amount of 580 to 590 mg to fill a size 0, hard-gelatin capsule.

## Claims

1. A compound represented by the following general structural formulae (I) or (II).

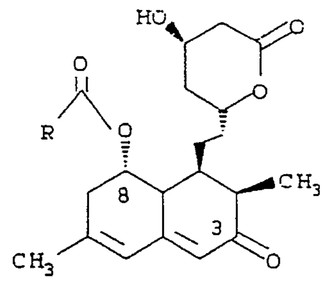

(I)

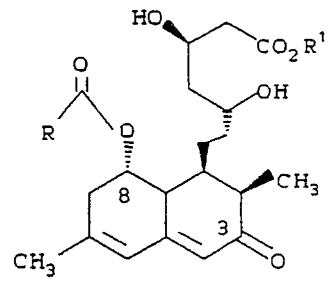

(II)

wherein:

R is $C_{1-10}$ alkyl; and

$R^1$ is hydrogen, $C_{1-5}$ alkyl or $C_{1-5}$ alkyl substituted with a member of the group consisting of phenyl, dimethylamino or acetylamino;

or a pharmaceutically acceptable salt of the compound (II) in which $R^1$ is hydrogen.

2. A compound of Claim 1 wherein R is sec -butyl or 1,1-dimethylpropyl.

3. A compound of Claim 1 which is 6(R)-[2-[8(S)-(2-methylbutyryloxy)-2(S),6-dimethyl-3-oxo-1,2,7,8,8a(R)-pentahydronaphthyl-1(S)]ethyl]-4(R)-hydroxy-3,4,5,6-tetrahydro-2H-pyran-2-one; or the ring opened dihydroxy acid analog or ester thereof.

4. A compound of Claim 1 which is 6(R)-[2-[8(S)-(2,2-dimethylbutyryloxy) 2(S),6-dimethyl-3-oxo-1,2,7,8,8a-(R)-pentahydronaphthyl-1(S)]-ethyl]-4(R)-hydroxy 3,4,5,6-tetrahydro-2H-pyran-2-one; or the ring opened dihydroxy acid analogs or ester thereof.

5. A hypocholesterolemic, hypolipidemic pharmaceutical composition comprising a nontoxic therapeutically effective amount of a compound of Claim 1 and a pharmaceutically acceptable carrier.

6. A composition of Claim 5 wherein the therapeutically active ingredient is 6(R)-[2-[8(S)-(2-methylbutyryloxy)-2(S),6-dimethyl-3-oxo-1,2,7,8,8a(R)-pentahydronaphthyl-1(S)]ethyl]-4(R)-hydroxy-3,4,5,6-tetrahydro-2H-pyran-2-one; or the ring opened dihydroxy acid analog or ester thereof.

7. A composition of Claim 5 wherein the therapeutically active ingredient is 6(R)-[2-[8(S)-(2,2-dimethylbutyryloxy)-2(S),6-dimethyl-3 oxo-1,2,7,8,8a(R)-pentahydronaphthyl-1(S)]ethyl]-4(R)-hydroxy-3,4,5,6-tetrahydro-2H-pyran-2-one; or the ring opened dihydroxy acid analog or ester thereof.

8. A hypocholesterolimic, hypolipidemic pharmaceutical composition comprising a nontoxic therapeutically effective amount of a compound of Claim 1 in combination with a pharmaceutically acceptable nontoxic cationic polymer capable of binding bile acids in a non-reabsorbable form in the gastrointestinal tract and a pharmaceutically acceptable carrier.

9. The use of a compound of Claim 1 for the preparation of a medicament useful for inhibiting cholesterol biosynthesis.

10. The use as claimed in Claim 9 wherein the therapeutically active ingredient is selected from:

(1) 6(R)-[2-[8(S)-(2-methylbutyryloxy)-2(S),6-dimethyl-3-oxo-1,2,7,8,8a(R)-pentahydronaphthyl-I(S)]-ethyl]-4(R)-hydroxy-3,4,5,6-tetrahydro-2H-pyran-2-one;

(2) 6(R)-2-[8(S)-(2,2-dimethylbutyryloxy)-2(S),6-dimethyl-3-oxo-1,2,7,8,8a(R)-pentahydronaphthyl-1(S)]-methyl-4(R)-hydroxy-3,4,5,6-tetrahydro-2H-pyran-2-one; or the ring opened dihydroxy acid analog or ester thereof.

European Patent Office

# EUROPEAN SEARCH REPORT

Application Number

EP 89 20 1884

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB-A-2 075 013 (SANKYO)<br>* Pages 1-3; claims *<br>----- | 1,5,8-10 | C 07 D 309/30<br>C 07 C 69/738<br>C 07 C 59/90<br>C 07 C 69/757 |
| | | | **TECHNICAL FIELDS SEARCHED (Int. Cl.5)** |
| | | | C 07 D 309/30<br>C 07 C |

The present search report has been drawn up for all claims

| Place of search | Date of completion of the search | Examiner |
|---|---|---|
| THE HAGUE | 08-02-1990 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)